# EUROPÄISCHE PATENTSCHRIFT

(11) **EP 0 921 807 B1**
(45) Veröffentlichungstag und Bekanntmachung des Hinweises auf die Patenterteilung: **05.11.2003**
(21) Anmeldenummer: 97944778.6
(22) Anmeldetag: 22.08.1997
(51) Int. Cl.: A61K 33/00, A61P 11/00, A61P 35/00

(54) **WASSERSTOFFHALTIGES MEDIKAMENT**
HYDROGENOUS MEDICAMENT
MEDICAMENT CONTENANT DE L'HYDROGENE

(30) Priorität: 27.08.1996 DE 19634530; 07.08.1997 DE 19734279
(43) Veröffentlichungstag der Anmeldung: 16.06.1999
(73) Patentinhaber: Messer Griesheim GmbH, 65933 Frankfurt am Main (DE)
(72) Erfinder: ESCHWEY, Manfred, D-40489 Düsseldorf (DE); KREBS, Christian, A-1120 Wien (AT); VAN BONN, Rolf, D-47269 Duisburg (DE); GERMANN, Peter, A-1190 Wien (AT)
(86) Internationale Anmeldenummer: EP9704567
(87) Internationale Veröffentlichungsnummer: WO98008523

(56) Entgegenhaltungen:
- DE-A- 2 836 770
- S.R. KAYAR ET AL.: "Hydrogen gas is not oxidized by mammalian tissues under hyperbaric conditions." UNDERSEA HYPERBARIC MED., Bd. 21, Nr. 3, 1994, Seiten 265-275, XP002052530

## Beschreibung

Die Erfindung betrifft wasserstoffgashaltige Medikamente, Verfahren zu deren Herstellung und deren Verwendung zur Herstellung eines Medikaments zur Behandlung von Lungenerkrankungen und entzündlichen Prozessen im Körper von Säugetieren und dem Menschen.

Die Verwendung von Stickstoffmonoxid (NO) enthaltenden Gasgemischen zur Behandlung von reversibler pulmonaler Vasokonstriktion und Bronchokonstriktion wird in WO 92/10228-A1 beschrieben. Das NO-Gasgemisch wird einem Beatmungsgas zugesetzt.

Im medizinischen Bereich werden Gase, zum Beispiel Lachgas (N₂O) und Xenon, zur Anaesthesie eingesetzt.

Eine Verwendung von Wasserstoffgas als Medikament ist nicht bekannt.

Der Erfindung liegt die Aufgabe zugrunde, ein Medikament zur Behandlung von entzündlichen Prozessen im Körper bereitzustellen.

Überraschenderweise wurde gefunden, daß die Verabreichung wasserstoffhaltiger Gasgemische einer Schädigung der Lunge von Säugetieren vorbeugt. Außerdem wird eine allgemeine entzündungshemmende oder antiinflammatorische Wirkung von wasserstoffhaltigen Gasgemischen beobachtet.
Gegenstand der Erfindung ist somit ein Medikament, das Wasserstoffgas oder eine Quelle für Wasserstoffgas enthält. Das Medikament dient zur Behandlung von Säugetieren und Mensch.

Der Begriff Wasserstoff (H) ist allgemein zu verstehen und umfaßt alle Isotope des Wasserstoffs, d. h. Protium (P oder ¹H), Deuterium (D oder ²H) und Tritium (T oder ³H). Molekularer Wasserstoff umfaßt daher alle möglichen Kombinationen der Wasserstoffisotope untereinander, d. h. P₂, PD, PT, DT, D₂ und T₂.

Wasserstoffgas enthält normalerweise molekularen Wasserstoff. Wasserstoffgas besteht beispielsweise aus P₂, PD oder D₂ oder Gemischen wie P₂/PD, PD/D₂, P₂/D₂, P₂/PD/D₂, wobei das normale Wasserstoffgas (H₂) mehr als 99 Vol.-% P₂ enthält. Das Wasserstoffgas kann auch atomaren Wasserstoff (Wasserstoff in statu nascendi) enthalten. Molekularer Wasserstoff existiert in den beiden Formen ortho-Wasserstoff (mit parallel orientierten Kernspins) und para-Wasserstoff (mit antiparallel orientierten Kernspins). Beide Formen stehen miteinander im thermischen Gleichgewicht. Oberhalb der Temperatur von 200 K liegt ein Gemisch konstanter Zusammensetzung aus 75 Vol.-% ortho- und 25 Vol.-% para-Wasserstoff vor. Wasserstoffgas dieser Zusammensetzung wird n-Wasserstoff genannt.

Die Gehaltsangaben (z. B. Vol.-%), Volumenangaben und Druckangaben beziehen sich auf Normalbedingungen (20° C. 1,013 bar). Die Einheit ppm wird definiert als ein Volumenteil pro 10⁶ Volumenteile. Der Begriff "wasserstoffgashaltiges Gasgemisch" umfaßt auch reines Wasserstoffgas.

Wasserstoffgas kommt in Stahlflaschen unter z. B. 200 bar in den Handel oder es wird flüssig (kryogen) bei -253° C in hochwärmeisolierten Behältern gelagert.

Das Medikament gemäß der Erfindung kann in unterschiedlicher Form eingesetzt werden. Das Medikament kann ein wasserstoffgashaltiges Gasgemisch oder eine flüssige (z. B. wasserstoffgashaltiges Gasgemisch oder reines Wasserstoffgas, dispergiert in einer Fettemulsion) oder feste Präparation (z. B. als eingelagertes Gas in Clathraten) sein.

Flüssige Medikamente werden bevorzugt intravenös systemisch oder topisch appliziert. Feste Medikamente oder Medikamente die Feststoffe enthalten, werden bevorzugt topisch (auf der Körperoberfläche) appliziert. Das Gasgemisch kann beispielsweise aus einem oder mehreren inerten Gasen und Wasserstoff oder aus einem oder mehreren inerten Gasen, Sauerstoff und Wasserstoff bestehen. Inerte Gase sind Stickstoff und Edelgase wie Helium, Neon, Argon, Krypton, Xenon und Radon.

Gasförmige Medikamente dienen bevorzugt zur Inhalation. Bei den gasförmigen Medikamenten ist das Medikament entweder ein atembares Gasgemisch, z. B. ein Wasserstoffgas-Gasgemisch mit 15 bis 30 Vol.-% Sauerstoff, oder das Medikament wird dem Atemgas (Beatmungsgas) zugemischt, z. B. wie in EP 0621 051-A2 beschrieben, worauf Bezug genommen wird. Ein inhalierbares Medikament wird beispielsweise durch Mischung von einem wasserstoffgashaltigen Gasgemisch, z. B. Wasserstoffgas in einem inerten Gas, mit Luft oder einem Beatmungsgas hergestellt. Ein inhalierbares Medikament, das Wasserstoffgas oder eine Quelle für Wasserstoffgas enthält, ist z. B. auch ein Aerosol.

Das Medikament oder eine Vorstufe zur Herstellung des Medikamentes enthält Wasserstoffgas in einer pharmakologisch wirksamen Menge. Eine pharmakologisch wirksame Menge von Wasserstoffgas im Gasgemisch liegt im allgemeinen im Bereich von 1 ppm bis 100 Vol.-%, vorteilhaft im Bereich von 100 ppm bis 80 Vol.-%, besonders vorteilhaft im Bereich von 0,1 Vol.-% bis 4 Vol.-% und insbesondere im Bereich von 1 bis 4 Vol.-% Wasserstoffgas. Die Zeit der Verabreichung von Wasserstoffgas, Reinstgas oder Gasgemisch oder wasserstoffgashaltigem Medikament, kann kurzzeitig, z. B. über Sekunden, Minuten oder Stunden, oder langzeitig, z. B. mehrere Tage oder gar Wochen, erfolgen.

Zur Herstellung des Medikamentes kann reines Wasserstoffgas verwendet werden. Aus Sicherheitsgründen oder aus Gründen einer einfacheren Dosierbarkeit werden zur Herstellung des Medikamentes vorteilhaft wasserstoffgashaltige Gasgemische verwendet. Ein zur Herstellung des Medikamentes geeignetes Gasgemisch ist beispielsweise ein Wasserstoff-Inertgas-Gemisch, z. B. 0,1 bis 10 Vol.-% Wasserstoff in Stickstoff. Zur Herstellung der wasserstoffgashaltigen Gasgemische oder Medikamente empfiehlt es sich, Gase sehr hoher Reinheit zu verwenden (z. B. Wasserstoffgas oder Stickstoffgas der Reinheit 6.0 oder höher). Die Ausgangsgase, die wasserstoffgashaltigen Gasgemische oder gasförmigen Medikamente, werden gewöhnlich komprimiert in Druckgasbehältern gespeichert Das Speichern des wasserstoffgashaltigen Gasgemisches in einem Druckgasbehälter ist insoweit von Vorteil, als daß damit eine bessere Dosierung möglich ist. Der Wasserstoff für das wasserstoffhaltige Gasgemisch kann auch durch Elektrolyse hergestellt oder bereitgestellt werden.

Wasserstoffgas ist in seiner Wirkung nicht auf die Gasphase beschränkt. Entsprechend dem Henry'schen Gesetz wird ein gewisser Anteil des Wasserstoffgases in Flüssigkeiten physikalisch gelöst. Demzufolge ist das Wasserstoffgas auch in wässrigen Lösungen (z. B. Körperflüssigkeit) wirksam.

Darüber hinaus kann Wasserstoffgas bedingt durch seine Löslichkeit in lipophilen Medien wirken. Gelöstes Wasserstoffgas entfaltet beispielsweise in und an Membranen biologischer Systeme seine heilende Wirkung, wozu neben der Zytoplasmamembran auch diejenigen des endoplasmatischen Reticulums, des Golgi-Apparates, der Lysosomen, der Kernmembran sowie der Mitochondrien zählen. Dies ist insoweit von besonderer Bedeutung, als daß an derartigen Membransystemen entscheidende biologische Reaktionen ablaufen, die sich unter anderem durch ihren gerichteten Charakter auszeichnen.

Neben der grundsätzlichen Möglichkeit, daß das erfindungsgemäße Medikament Wasserstoffgas bereits in Gasform enthält, kann das Medikament auch eine Quelle für Wasserstoffgas enthalten. Bei Quellen für Wasserstoffgas liegt der Wasserstoff in chemisch oder physikalisch gebundener Form vor, aus denen Wasserstoffgas zum erwünschten Zeitpunkt freigesetzt wird (z. B. wasserstoffabspaltende Verbindungen, Metallhydride, Clathrate, Mikropartikel).

Wasserstoffgashaltige Medikamente dienen zur Behandlung von entzündlichen Prozessen im Körper, z. B. Entzündungen von Organen (z. B. Lunge) oder Entzündungen an Haut oder Schleimhaut. Wasserstoffgashaltige Medikamente eignen sich insbesondere zur Behandlung von entzündlichen Prozessen in der Lunge, das sind in der Regel alle Prozesse, die mit einer vasokonstriktorischen oder bronchokonstriktorischen Wirkung einhergehen. Wasserstoffgashaltige Medikamente, insbesondere inhalierbare wasserstoffgashaltige Medikamente, eignen zur Behandlung und Prävention folgender Krankheiten: ARDS (Adult Respiratory Distress Syndrome), Asthma bronchiale, COPD (chronisch obstruktive Atemwegserkrankung; Chronic Obstructive Pulmonary Disease), Bronchitis, Pneumonie, Lungentrauma, hypoxisch bedingte Vasokonstriktion.

Ohne an die Feststellung gebunden zu sein, scheint nach heutigem Erkenntnisstand die pharmakologische Wirkung von Wasserstoffgas unter physiologischen Bedingungen u.a. darauf zu beruhen, daß bei entzündlichen Prozessen eine erhöhte Ausschüttung reaktiver Spezies wie Radikale (Peroxynitrit, Hydroxylradikal) erfolgt, die durch Reaktion mit Wasserstoffgas abgefangen werden. Insbesondere die Reaktion von Wasserstoffgas mit endogen gebildetem Peroxynitrit und Hydroxylradikalen scheint wesentlich für die pharmakologische Wirkung von Wasserstoffgas zu sein.

Überraschend wurde eine toxische Wirkung von deuteriumhaltigen Gasgemischen auf Tumorzellen gefunden. Deuteriumhaltige Gasgemische, d. h. Gasgemische mit D, D₂ oder PD, können daher zur Behandlung von Tumoren oder Krebs eingesetzt werden. Deuteriumhaltige Gasgemische eignen sich insbesondere als inhalierbares Medikament zur Behandlung oder Prävention von Lungenkrebs oder zur Unterstützung der Behandlung von Lungenkrebs in Kombination mit anderen Medikamenten. Zur inhalativen Therapie kann beispielsweise ein Beatmungsgas mit 0,1 bis 4 Vol.-% D₂, PD oder deren Gemisch, 20 bis 30 Vol.-% Sauerstoff und Stickstoff als restlichem Gas verwendet werden. Ein deuteriumhaltiges Gasgemisch kann auch H₂ enthalten. Ein solches Medikament vereint die günstigen pharmakologischen Wirkungen von deuteriumhaltigen Gas und Wasserstoffgas. Das Medikament kann beispielsweise ein Beatmungsgas mit 0,1 bis 4 Vol.-% D₂, PD oder deren Gemisch und mit 0,1 bis 4 Vol.-% H₂ sein, wobei deuteriumhaltiges Gas und Wasserstoffgas einen Anteil von zusammen 4 Vol.-% nicht übersteigen, 20 bis 30 Vol.-% Sauerstoff und Stickstoff als restlichem Gas.
Zur Prävention von Lungenkrebs kann beispielsweise ein Beatmungsgas mit 10 bis 1000 ppm, insbesondere 10 bis 100 ppm D₂, PD oder deren Gemisch verwendet werden. Beispielsweise bei starken Rauchern kann ein mobiles Gerät mit deuteriumhaltigen Gas zur Beimischung zum Atemgas eingesetzt werden. Beatmungsgase werden in der Regel vor der Inspiration befeuchtet.

Hinsichtlich der Dosierung von Wasserstoffgas in entsprechenden Gasgemischen sind die entsprechenden Zünd- bzw. Explosionsgrenzen zu berücksichtigen, die sich in Abhängigkeit von der Menge des enthaltenen Sauerstoffs sowie der Feuchtigeit der Gase, wie dem Fachmann bekannt ist, ändern.

Die vorteilhafte pharmakologische Wirkung von wasserstoffhaltigen Gasgemischen kann auch bei Zell-, Gewebe- und Organkulturen angewendet werden. Beispielsweise können Zellkulturen mit wasserstoffhaltigen Gasgemischen begast werden. Es können aber auch flüssige, gelartige oder feste wasserstoffgashaltige Präparationen oder Quellen für Wasserstoffgas als Bestandteile oder Zusätze des Nährbodens oder von Nährlösungen für Kulturen von Zellen, Mikroorganismen, Geweben oder Organen verwendet werden.

Die Sauerstoffaufnahme in der Lunge kann durch Behandlung mit einem wasserstoffgashaltigen Gasgemisch als Medikament verbessert werden.
Durch die geringe Molekülgröße von Wasserstoff, und damit einhergehend günstiger Transporteigenschaften, ausgedrückt in niedriger Viskosität, hohem Kriech- und Diffusionsvermögen, wird insbesondere bei Verschlüssen des Atemweges (Vorliegen von Atelektasen) die Beatmung und der damit verbundene Stoffaustausch verbessert. Ohne sich darauf festzulegen, scheint der verbesserte Stoffaustausch damit in Zusammenhang zu stehen, daß Sauerstoffmoleküle im Sog der Wasserstoffmoleküle mitgerissen werden.

Die Beatmung kann durch Zumischen von Helium zu dem wasserstoffgashaltigen Gasgemisch weiter erhöht werden. Ein Gasgemisch mit Wasserstoffgas, Helium und Sauerstoffgas dient daher zur Herstellung eines inhalierbaren Medikamentes zur Behandlung eines gestörten Gasaustausches in der Lunge und zur Behandlung von Verschlüssen der Atemwege. Ein solches inhalierbare Medikament oder Beatmungsgas enthält beispielsweise 0,1 bis 4 Vol.-% Wasserstoffgas, 0,1 bis 60 Vol.-% Helium, 20 bis 35 Vol.-% Sauerstoff und Rest Stickstoff, wobei alle Anteile zusammen 100 Vol.-% ergeben, oder 0,1 bis 4 Vol.-% Wasserstoffgas, 20 bis 35 Vol.-% Sauerstoff und Rest Helium (zusammen 100 Vol.-%). Zur Verbesserung des Stoffaustausches und zur Behandlung von Verschlüssen der Atemwege werden bevorzugt wasserstoffgashaltige Gasgemische als Medikament verwendet mit einer Zusammensetzung von 1 bis 4 Vol.-% Wasserstoffgas, 1 bis 50 Vol.-% Helium, 20 bis 30 Vol.-% Sauerstoff und Rest Stickstoff (zusammen 100 Vol.-%) oder 1 bis 4 Vol.-% Wasserstoffgas, 20 bis 30 Vol.-% Sauerstoff und Rest Helium (zusammen 100 Vol.-%), insbesondere 3 bis 4 Vol.-% Wasserstoffgas, 10 bis 50 Vol.-% Helium, 20 bis 30 Vol.-% Sauerstoff und Rest Stickstoff (zusammen 100 Vol.-%) oder 3 bis 4 Vol.-% Wasserstoffgas, 20 bis 30 Vol.-% Sauerstoff und Rest Helium (zusammen 100 Vol.-%).

Das wasserstoffgashaltige Gasgemisch kann ein pharmakologisch wirksames Gas enthalten, z. B. NO (Stickstoffmonoxid), N₂O (Distickstoffoxid), Acetylen (Ethin; C₂H₂), Ethylen (Ethen; C₂H₄) oder Kohlenmonoxid (CO). Die Konzentration des pharmakologisch wirksamen Gases kann im Bereich von 1 ppm bis 99 Vol.-%, bevorzugt im Bereich von 1 ppm bis 80 Vol.-% und besonders bevorzugt im Bereich von 1 ppm bis 50 Vol.-%, liegen. Für gasförmige Medikamente, die zur Inhalation bestimmt sind, liegt die Konzentration von NO oder CO im wasserstoffgashaltigen Gasgemisch im allgemeinen im Bereich von 1 bis 1000 ppm, bevorzugt 1 bis 500 ppm und besonders bevorzugt 50 bis 400 ppm. N₂O, Ethylen und Acetylen können beispielsweise in Konzentrationen im Bereich von 1 ppm bis 80 Vol.-%, bevorzugt 1 ppm bis 50 Vol.-% und besonders bevorzugt 1 ppm bis 2,5 Vol.-%, in den Medikamenten zur Inhalation enthalten sein.

Überraschend wurde gefunden, daß Gasgemische mit Wasserstoffgas und NO sich nicht nur in vorteilhafterweise in ihrer Wirkung ergänzen, sondern zusätzlich die Wirkung von NO bei der Inhalationstherapie von Lungenerkrankungen gesteigert wird. Als pharmakologisch wirksames Gas für den Zusatz zu einem wasserstoffgashaltigen Gasgemisch wird NO daher besonders bevorzugt. Die pharmakologische Wirkung von NO, das auch endogen gebildet wird, wird in "Nüssler, A.K.; PZ, Nr. 2, 141. Jahrgang, Ausgabe vom 11.1.1996; S. 11-20" beschrieben, worauf Bezug genommen wird. Das eingesetzte NO stammt im allgemeinen aus einem Gasgemisch als NO-Quelle, das z. B. als Gemisch von 900 ppm NO in Stickstoff (für medizinische Zwecke) in Druckgasflaschen im Handel erhältlich ist.

Als NO-Quelle sind auch Substanzen geeignet, die NO abgeben (NO-Donatoren), z. B. S-Nitroso-N-acetyl-penicillamin, S-Nitroso-cystein, Nitroprussid, Nitroguanidin, Glyceryl-trinitrat, Isoamyl-nitrit, anorganisches Nitrit, Azid und Hydroxylamine.

Wasserstoffhaltige Gasgemische mit NO oder einer NO-Quelle dienen zur Herstellung eines Medikamentes zur Behandlung von reversibler und irreversibler pulmonaler Vasokonstriktion (Lungengefäßkrampf), Bronchokonstriktion (krampfartige Verengung der Bronchien) und entzündlichen Prozessen der Lunge, insbesondere zur Behandlung oder Prävention der Krankheiten Pneumonie (Lungenentzündung), Lungentrauma, Asthma bronchiale, ARDS (schwere Schocklunge; Acute Respiratory Distress Syndrome), PPHN (angeborener Hochdruck im arteriellen Lungenstromgebiet; Persistent Pulmonary Hypertension of the Newborn), COPD (chronisch obstruktive Atemwegserkrankung; Chronic Obstructive Pulmonary Disease), Bronchitis (Entzündung der Bronchien), hypoxisch bedingte Vasokonstriktion (Gefäßkrampf durch Sauerstoffmangel), Fettembolie in der Lunge (Verstopfung der Lungenarterien), akute pulmonale Ödeme (akute Flüssigkeitseinlagerung in der Lunge), akute Höhenerkrankung (acute mountain sickness), erhöhter Lungenhochdruck nach herzchirurgischen Eingriffen (postcardiac surgery acute pulmonary hypertension), Aspirationssyndrom der Neugeborenen (perinatal aspiration syndrome), hyaline Membranerkrankung (hyaline membrane disease), akute Lungenembolie (acute pulmonary thromboembolism), Heparin-Protamin-Reaktionen, Sepsis, Asthmaanfall (status asthmaticus), Sauerstoffunterversorgung (hypoxia), chronischer Lungenhochdruck (chronic pulmonary hypertension), Fehlentwicklung der Lunge/Bronchien (bronchopulmonary dysplasia), chronische Lungenembolie (chronic pulmonary thromboembolism), idiopathischer oder primärer Lungenhochdruck (idiopathic pulmonary hypertension or primary pulmonary hypertension) oder chronische Sauerstoffunterversorgung (chronic hypoxia).

Statt der simultanen Verabreichung von Wasserstoffgas und NO in einem Gasgemisch bei der Inhalationstherapie kann eine getrennte Verabreichung von Wasserstoffgas und NO von Vorteil sein. Beispielsweise kann ein wasserstoffgashaltiges Gas und ein NO-haltiges Gas unabhängig voneinander in ein Beatmungsgas dosiert werden, was eine jeweils therapeutisch angepaßte Dosierung der einzelnen Gase im zeitlichen Verlauf der Behandlung ermöglicht. Je nach Reaktion des Körpers eines Patienten kann also die Wasserstoffgas-Menge und die NO-Menge im Beatmungsgas variiert werden.

Hinsichtlich der zeitlichen Kombinationen der Verabreichung von wasserstoffgashaltigem Gasgemisch und NO-haltigem Gas oder einer inhalierbaren NO-Quelle sind grundsätzlich alle Kombinationen einsetzbar.
So kann beispielsweise zuerst NO dem Beatmungsgas zugeführt werden und erst danach Wasserstoffgas oder umgekehrt.

Im folgenden sind unter den Begriffen "wasserstoffgashaltige Gasgemische" oder "wasserstoffgashaltig" Gasgemische oder Zusammensetzungen zu verstehen, die Wasserstoffgas (rein oder im Gemisch), Wasserstoffgas und NO oder Wasserstoffgas und ein pharmakologisch wirksames Gas enthalten.

Zur Herstellung flüssiger pharmazeutischer Zusammensetzungen (Medikamente) kann das wasserstoffgashaltige Gasgemisch in einer wäßrigen Phase unter Normaldruck oder unter Druck gelöst oder dispergiert werden. Die wäßrige Phase kann Zucker, Zuckeralkohole, Dextrose, Dextrane, Polysacchararide (z. B. Stärke, Cellulose, Amylose, Pektin, Agar), derivatisierte Polysaccharide (z. B. Methylcellulose), Proteine (z. B. Albumin), Tenside und/oder Salze enthalten. Wäßrige, gasenthaltende Zusammensetzungen werden in EP 0122624-A1 und EP 0123235-A1 beschrieben, worauf Bezug genommen wird. Die Viskosität der wäßrigen Phase kann durch Zusätze (z. B. Polyethylenglykol, Methylcellulose etc.) eingestellt werden. Die wäßrige Phase ist bevorzugt zur Infusion geeignet und genügt physiologischen Bedingungen (isotone Lösungen). wäßrige Zusammensetzungen mit Kohlenhydraten (z. B. Zucker, Polysacchararide) und/oder Protein oder Proteingemischen (z. B. Albumin) können auch für die Herstellung von wasserstoffgashaltigen Mikrobläschen (Microbubbles) verwendet werden. Die Herstellung von gashaltigen Mikrobläschen wird in WO 96/38181-A1 beschrieben, worauf Bezug genommen wird. Wäßrige Zusammensetzungen mit wasserstoffgashaltigen Mikrobläschen erhält man beispielsweise durch Ultraschallbehandlung einer Lösung von 1 Teil einer wäßrigen Lösung mit 5 Gew.-% Humanalbumin und 3 Teilen einer Lösung mit 5 Gew.-% Dextrose unter einer wasserstoffgashaltigen Atmosphäre. Die gashaltigen Mikrobläschen haben in der Regel einen Durchmesser von 1 bis 10 Mikrometer.

Die Herstellung von gashaltigen Mikrotröpfchen (Microdroplets) wird in US 4622219 beschrieben, worauf bezug genommen wird.

Flüssige oder gelartige pharmazeutische Zusammensetzungen können auch dadurch hergestellt werden, daß das wasserstoffgashaltige Gasgemisch in einer lipophilen Phase unter Normaldruck oder bevorzugt unter Druck gelöst oder dispergiert wird.

Die lipophile Phase enthält als lipophile Bestandteile ein Alkan oder Alkan-Gemisch (z. B. Heptan, höhere Alkane, Mineralöl), pflanzliche oder tierische Öle (z. B. Olivenöl, Baumwollsamenöl, Sojabohnenöl, Distelöl, Fischöl), Ether (z. B. Dipropylether, Dibutylether), Ester (z. B. langkettige Ester oder hydrophobe Ester), Silikone, Fluorkohlenstoffverbindungen (z. B. Perfluorpolyether, Perfluordecalin, Perfluortripropylamin, Perfluormethyl-adamantan, Blutersatzstoffe, Perfluorbromalkylether, Perfluorhexylether, Perfluorbutylethen, Perfluorisopropylhexylethen, Perfluoroctylbromid, die allgemeinen Klassen Perfluoralkylether, Perfluoralkylalkene, Perfluoralkylarylether, Perfluoralkylarylalkene, Perfluorarylether, Perfluorarylalkene), ein oder mehrere Lipide (z. B. Kohlenwasserstoffe wie Tricontan, Squalen, Carotinoide; Alkohole wie Wachsalkohole, Retinol, Cholesterin; Ether; Carbonsäuren wie Fettsäuren; Ester (Neutralfette, Mono-,Di-, Triacylglycerine, Wachse, Stearinsäureester); Amide (Ceramide); Glykolipide oder Phospholipide (z. B. Phosphatidylcholin, Phosphatidylethanolamin, Phosphatidylserin, Phosphatidylglycerin, Phosphatidylinositol, Lecithin). Die lipophile Phase kann insbesondere Liposomen enthalten oder aus Liposomen bestehen. Die lipophile Phase enthält bevorzugt ein Tensid. Die lipophile, wasserstoffgashaltige Phase kann direkt als Medikament eingesetzt werden oder mit einer hydrophilen Phase gemischt werden (Bildung einer Dispersion, Emulsion oder Suspension). Die hydrophile Phase kann Wasser und/oder ein hydrophiles Lösemittel wie Ethanol, Glycerin, oder Polyethylenglykol enthalten. Die Emulsion enthält vorzugsweise ein oder mehrere Tenside und/oder einen oder mehrere Emulgatoren. Geeignete Emulgatoren sind z. B. Sojaphosphatide, Gelatine oder Eiphosphatid. Die Emulsionen oder Suspensionen enthalten z. B. 10 bis 30 Gew.-% lipophile Phase. Geignete Fettemulsionen sind kommerziell erhältlich, z. B. von der Firma Pharmacia & Upjohn, Erlangen, die Produkte Intralipid^{(R)}10 (100g Sojabohnenöl, 6g Phosphatidylcholin, 22g Glycerin, ad 1000ml Wasser) und Intralipid^{(R)}20 (200g Sojabohnenöl, 12g Phosphatidylcholin, 22g Glycerin, ad 1000ml Wasser). Die Mischungen aus lipophiler und hydrophiler Phase (z. B. Fettemulsion) werden mit dem Gas versehen beispielsweise durch Hindurchleiten oder Hindurchperlen des Gases durch die Mischung bei Normaldruck oder Druck (z. B. 1-300 bar) bei einer Temperatur von beispielsweise 0° C bis 50° C. Vorzugsweise wird die wasserstoffgashaltige Mischung im Hochdruckautoklaven durch Zusammengeben der Mischung und des Gases bei einem Druck von 100 bis 300 bar hergestellt. Es kann auch erst die lipophile Phase mit wasserstoffgashaltigem Gasgemisch beladen werden und dann die Mischung mit der hydrophilen Phase erfolgen.

Flüssige oder gelartige pharmazeutische Zusammensetzungen können wasserstoffgashaltige Liposomen oder wasserstoffgashaltige Liposomen-haltige Flüssigkeiten sein. Wasserstoffgashaltige Liposomen können nach üblichen Methoden hergestellt werden. Die Herstellung gashaltiger Liposomen ist in US 5334381 beschrieben, worauf Bezug genommen wird. Die pharmazeutischen Zusammensetzungen mit wasserstoffgashaltigen Liposomen werden im allgemeinen als injizierbare Medikamente eingesetzt. Es können auch Salben mit wasserstoffgashaltigen Liposomen hergestellt werden. Die Salben werden zum Beispiel äußerlich eingesetzt.

Zur Herstellung von wasserstoffgashaltigen Lösungen oder Emulsionen, insbesondere Liposomen, können übliche Methoden wie Ultraschallverfahren oder mechanische Homogenisierungsverfahren eingesetzt werden. Als Homogenisiergerät kann beispielsweise das Gerät Ultra-Turrax^{(R)} der Firma Jahnke & Kunkel verwendet werden. Flüssige wasserstoffgashaltige Zusammensetzungen können auch durch Hindurchperlen (z. B. Feinverteilungsfritte in Gaswaschflasche) durch die Flüssigkeit hergestellt werden. Die Beladung mit wasserstoffgashaltigem Gas erfolgt vorzugsweise unter Druck.

Flüssige oder gelartige pharmazeutische Zusammensetzungen können wasserstoffgashaltige Mikropartikel enthalten. Die Mikropartikel haben im allgemeinen einen Partikeldurchmesser von 0,1 bis 40 Mikrometer. Die Mikropartikel sind beispielsweise aus Polymeren aufgebaut und enthalten in einer Polymerhülle das Gas. Die Polymere sind vorzugsweise bioabbaubar. Geeignete Polymere sind beispielsweise Homopolyaldehyde oder Copolyaldehyde mit Molekulargewichten im Bereich von 1000 bis 12000 dalton. Geeignete Monomere sind z. B. alpha, betaungesättigte Aldehyde wie Acrolein und Glutaraldehyd. Die Aldehyd-Funktionen (Formylgruppen) der Mikropartikel eignen sich direkt oder nach Umwandlung in andere funktionelle Gruppen (z. B. Hydroxy- oder Carboxyl-Gruppe) zur Derivatisierung mit den unterschiedlichsten Kopplungsagenzien, z. B. Hydroxylamin, Trishydroxymethylaminomethan, 3-Amino-1-propansulfonsäure, D-Glukosaminhydrochlorid, Aminomannit, Harnstoff, Humanalbumin, Hydrazin, Peptide, Proteine, Polyglykolamine, Aminopolyalkohole (z. B. HO-PEG-NH₂ oder NH₂-PEG- NH₂; PEG: Polyethylenglykol) oder säuregruppenhaltige Verbindungen (z. B. PEG-Linker-Glutaminsäure, PEG-Linker-DTPA oder PEG-Linker-EDTA; PEG: Polyethylenglykol). PEG-Gruppen haben in der Regel ein Molekulargewicht unter 100000 dalton, vorzugsweise unter 40000 dalton. Die Herstellung und Formulierung gashaltiger Mikropartikel wird in EP 0 441 468-B1 beschrieben, worauf hiermit Bezug genommen wird. Die wasserstoffgashaltigen Mikropartikel können in den verschiedensten galenischen Formulierungen eingesetzt werden (z. B. 50 mg wasserstoffgashaltige Mikropartikel, 860 mg Natriumchlorid, ad 100 ml Wasser). Die pharmazeutischen Zusammensetzungen enthalten beispielsweise 0,1 Mikrogramm bis 100 mg Mikropartikel/ml, vorzugsweise 10 Mikrogramm bis 10 mg Mikropartikel/ml.

Cavitate oder Clathrate von Gasen werden als Ultraschallkontrastmittel eingesetzt. Ihre Herstellung ist z. B. in EP 0 357 163-A1 beschrieben, worauf Bezug genommen wird. Pharmazeutische Zusammensetzungen mit Cavitaten oder Clathraten von wasserstoffgashaltigen Gasgemischen bestehen beispielsweise aus Hydrochinon, Harnstoff oder Thioharnstoff als sogenannte Wirtsmoleküle (Wirtssubstanz) und Wasserstoffgas als Gastmoleküle. Die Herstellung der Clathrate erfolgt im allgemeinen mit Lösungen der Wirtssubstanz in einem Lösemittel wie Ethanol oder Propanol, wobei die heiße Lösung (z. B. 60, 70° C oder höher, je nach Wirtssubstanz) in einen Hochdruckautoklaven gebracht wird und die Lösung mit dem wasserstoffhaltigen Gas bei hohem Druck (z. B. 150 bis 300 bar) beaufschlagt wird.

Der Hochdruckautoklav wird dann gewöhnlich für einige Zeit temperiert (z. B. 80° C für 2 Stunden). Danach wird der Hochdruckautoklav allmählich abgekühlt (z. B. über 5 Tage). Die ausfallenden Kristalle werden dann abgetrennt und im allgemeinen mit dem Lösemittel gewaschen. Die Korngröße der Kristalle kann durch Herstellungsbedingungen oder durch mechanische Verfahren der Partikelzerkleinerung variiert werden. Die kristallinen Clathrate können mit hydrophilen, lipophilen oder amphiphilen Hilfsstoffen überzogen werden. Zur Applikation werden die Clathrate vorteilhaft in einem sterilen wäßrigen System mit Zusätzen zur Einstellung der Viskosität, Oberflächenspannung, pH-Wert und osmotischem Druck aufgenommen (z. B. suspendiert). Für Hydrochinon als Wirtssubstanz eignet sich beispielsweise ein wäßriges System mit folgender Zusammensetzung: 1 % Gelatinelösung, 1 % Albuminlösung, 10 % Glycerinlösung, 15 % Propylenglykollösung, Mischungen von Natriumcholat und Phosphatidylcholin in Wasser, 0,01-1 % Phosphatidylcholindispersion (wäßrig), 1 % Methylcellulose, 1-2 % Dextranlösung, 1 % Agarlösung, 2 % Tweenlösung (Tween 80) und 1 % Gummi arabicum.

Durch die Auflösung des Clathrats wird das enthaltene Gas freigesetzt. Die Geschwindigkeit der Freisetzung des Gases ist unter anderem abhängig von der Wirtssubstanz, der Korngröße und dem verwendeten wäßrigen System und kann in weiten Grenzen eingestellt werden. Mit Hilfe der Clathrate sind auf einfachem Wege injizierbare, wasserstoffgashaltige pharmazeutische Zubereitungen zu erhalten.

10 mg des Hydrochinon/H₂-Komplexes (3:1 Komplex) setzen beispielsweise insgesamt um 0,03 mg H₂ frei.

Wasserstoffgashaltige Medikamente (z. B. mit oder ohne NO, CO, N₂O, Acetylen oder Ethylen) werden u.a. in Form von Zäpfchen, Salben, Lösungen, Dispersionen, Emulsionen, Mikrotröpfchen (Microdroplets), Mikrobläschen (Microbubbles), Liposomen, Mikropartikeln, Aerosolen, Schäumen, teilchenförmigen Mitteln, Pillen, Pastillen, Kapseln, Mikrokapseln, Kaugummiarten, in Trägern (Carrier) oder als Bestandteil von einem Pflaster angewendet.

Gasförmige Medikamente können oberflächlich (topisch) z. B. mit glockenförmigen Behältern drucklos oder mit Druck appliziert werden.

### Beispiel

### Beatmung mit Wasserstoffgas zur Prophylaxe

In einem Versuch mit Schafen wurde die prophylaktische Wirkung von Wasserstoffgas im Beatmungsgas untersucht. Ein künstliches Lungenversagen (ARDS) wurde mittels Ölsäure provoziert. Das normale Beatmungsgas bestand aus 50 Vol.-% Sauerstoff und Stickstoff als Rest. Das wasserstoffgashaltige Beatmungsgas war ein Gasgemisch aus 50 Vol.-% Sauerstoff, 3,6 Vol.-% Wasserstoffgas und Rest Stickstoff. Das Beatmungsgas wurde mittels einem Beatmungsgerät verabreicht, dabei wurde FiO₂ = 0,5 konstant (FiO₂: fraction of inspiratory oxygen) gehalten (eine stufenlose Variierung des Sauerstoff-Anteiles von 21-100 % ist möglich).

Die Tiere wurden 2 Tage vor dem Experiment instrumentiert, d. h. es wurden unter Narkose zentralvenöse Katheter zur Medikament- und Flüssigkeitsgabe, sowie das invasive Monitoring eingesetzt. Das invasive Monitoring umfaßte Messung des Herzzeitvolumens (Pumpleistung des Herzens in l/min., abgekürzt CO) mittels eines Swan-Ganz-Katheters, das auf dem Prinzip der Thermodilution arbeitet, der Messung des Druckes in der oberen Hohlvene vor dem rechten Herzvorhof, der Pulmonalarterie und einer peripheren Arterie. Die Bestimmung des Lungenwassergehaltes erfolgte mittels einer fiberoptischen Sonde in der Arteria femoralis (COLD, Fa. Pulsion, München, Deutschland).

Während des Experimentes waren die Versuchstiere, nach Narkoseeinleitung mit einem Barbiturat (Thiopental), mit einem Inhalationsnarkotikum (Isofluran) anästhesiert. Beatmet wurden die Tiere mittels druckkontrollierter Beatmung (Servo SV 900, Siemens, Deutschland).

Über einen sogenannten Scheibengasmischer, welcher normalerweise für die Anaesthesie mit N₂O verwendet wird, wurde das Gasgemisch mit 3,5 Vol.-% H₂ und Rest Stickstoff (Druckgasflasche; Messer Griesheim) in den Respirationskreis des Testtieres gebracht. Über den Dichteumrechnungsfaktor sowie einer außen am Scheibengasmischer angebrachten Skalierung konnte eine exakte H₂-Konzentration appliziert werden. Die H₂-Konzentration des Inspirationsgases (eingeatmetes Gas) und des Exspirationsgases (ausgeatmetes Gas) wurde überwacht. Als H₂-Meßgeräte wurden elektrosensorische Meßgeräte (Fa. Compur) und nach dem Wärmeleitprinzip arbeitende Meßgeräte (Hydros, Fa. Rosemount) verwendet. Die Beatmungsparameter wurden nach einmal fixierten Wertbestimmungen während der Behandlung nicht mehr verstellt.

Die Behandlung mit wasserstoffgashaltigem Beatmungsgas dauerte 60 Minuten. Danach wurde das Lungenversagen mit Ölsäure (siehe: Clinical Lessons from the Oleic Acid Model of Acute Lung Injury, Daniel P. Schuster, Am. J. Respir. Crit. Care Med., Vol. 149, 1994, Seite 245-260) induziert.

Die Ergebnisse sind in der Tabelle zusammengestellt.

Tabelle: Meßergebnisse bei prophylaktischer Wasserstoffgasbehandlung vor Einleitung eines künstlichen Lungenversagens bei einem Schaf und einem Schaf mit induziertem Lungenversagen ohne Wasserstoffgasbehandlung im Vergleich zu ARDS-Ausgangswerten der Schafe
(Es bedeuten: paO2: Sauerstoff-Partialdruck im Beatmungsgas (partial oxygen pressure); paCO2: Kohlendioxid-Partialdruck im Beatmungsgas (partial carbon dioxide pressure); SaO2: Sauerstoffsättigung im Blut (saturation of oxygen); PAPmean : mittlerer pulmonaler Arteriendruck (mean pulmonary arterial pressure); CO: Pumpleistung des Herzens (cardiac output);
Zahlenwerte mit Vorzeichen sind relative Abweichungen, Zahlenwerte in Klammern sind Absolutwerte)

| Parameter | ARDS-Ausgangswerte nach Ölsäure | Schaf mit H₂-Vorbehandlung und induziertem ARDS | Schaf ohne H₂-Vorbehandlung und induziertem ARDS |
|---|---|---|---|
| paO2 / mm Hg | (54) | +20 (65) | +12 (60) |
| paCO2 / mm Hg | (48) | -10 (43) | -5 (46) |
| paO2/paCO2 | (134) | +50 (201) | +35 (187) |
| SaO2 / % | (78) | +15 (90) | +8 (84) |
| Lymphfluß / ml/h | (32,5) | +18 (34) | +20 (39) |
| Lungenwasser / ml/kg | (15) | -6 (14) | +/- 0 (15) |
| PAPmean / mm Hg | (15) | + 5 (14) | -8 (14) |
| CO / l/min. | (4,5) | +3 (4,6) | -15 (3,8) |

Aufgrund der Ergebnisse in der Tabelle kann für die Behandlung mit Wasserstoffgas auf eine protektive Wirkung bei einer direkten Schädigung der Lunge, hervorgerufen durch Ölsäure, geschlossen werden. Die Applikation von Ölsäure führt über die Aktivierung verschiedener Entzündungsmediatoren zu einer Freisetzung von Sauerstoffradikalen, die zu einer toxischen Gewebsschädigung des Lungengewebes führen. Durch diese Schädigung der Zellbarriere kommt es zu einer vermehrten Flüssigkeitseinlagerung in das interstitielle und alveolare Gewebe der Lunge, was zu einer schweren Gasaustauschstörung führt. Die Versuchsergebnisse zeigen für die Behandlung mit Wasserstoffgas eine Reduktion der Gasaustauschstörung und eine Zunahme des Lymphflusses sowie eine Abnahme des Lungenwassers, was für eine Protektion der Zellbarriere spricht.

## Patentansprüche

1. Medikament, enthaltend Wasserstoffgas oder eine Quelle für Wasserstoffgas.

2. Medikament nach Anspruch 1, **dadurch gekennzeichnet, daß** es gasförmig, flüssig, gelartig oder fest vorliegt.

3. Medikament nach Anspruch 1 oder 2, **dadurch gekennzeichnet, daß** das Wasserstoffgas ein oder mehrere der Wasserstoffisotope Protium, Deuterium oder Tritium enthält.

4. Medikament nach einem der Ansprüche 1 bis 3, enthaltend Stickstoffmonoxid, Kohlenmonoxid, Distickstoffoxid, Acetylen oder Ethylen.

5. Medikament nach einem der Ansprüche 1 bis 4, enthaltend ein wasserstoffgashaltiges Gasgemisch mit 1ppm (v/v) bis 99 Vol.-% Stickstoffmonoxid, Kohlenmonoxid, Distickstoffoxid, Acetylen oder Ethylen.

6. Medikament nach einem der Ansprüche 1 bis 5, **dadurch gekennzeichnet, daß** das wasserstoffgashaltige Medikament als inhalierbares Gas, in Form von Zäpfchen, Salben, Lösungen, Dispersionen, Emulsionen, Mikrotröpfchen, Mikrobläschen, Liposomen, Mikropartikeln, Aerosolen, Schäumen, teilchenförmigen Mitteln, Pillen, Pastillen, Kapseln, Mikrokapseln, Kaugummiarten, in Trägem oder als Bestandteil von einem Pflaster angewendet wird.

7. Medikament nach einem der Ansprüche 1 bis 5, **dadurch gekennzeichnet, daß** wasserstoffgashaltige oder Waserstoffgas und ein oder mehrere der Gase Stickstoffmonoxid, Kohlenmonoxid, Distickstoffoxid, Acetylen oder Ethylen enthaltende Mikrotröpfchen, Mikrobläschen, Liposomen, Mikropartikel oder Clathrate im Medikament enthalten sind.

8. Verwendung einer wasserstoffgashaltigen Zusammensetzung oder Gasgemisches zur Herstellung eines Medikamentes zur Behandlung oder Prävention von ARDS (Adult Respiratory Distress Syndrome), Asthma bronchiale, COPD (chronisch obstruktive Atemwegserkrankung; Chronic Obstructive Pulmonary Disease), Bronchitis, Pneumonie, Lungentrauma, hypoxisch bedingte Vasokonstriktion, zur Behandlung von Verschlüssen der Atemwege (Atelektasen) oder entzündlichen Prozessen.

9. Verwendung einer wasserstoffgashaltigen und stickstoffmonoxidhaltigen Zusammensetzung oder Gasgemisches zur Herstellung eines Medikamentes zur Behandlung oder Prävention von pulmonaler Vasokonstriktion, Bronchokonstriktion oder Verschlüssen der Atemwege.

10. Verwendung einer wasserstoffgashaltigen und stickstoffmonoxidhaltigen Zusammensetzung zur Herstellung eines Medikamentes zur Behandlung von reversibler und irreversibler pulmonaler Vasokonstriktion (Lungengefäßkrampf), Bronchokonstriktion (krampfartige Verengung der Bronchien) und entzündlichen Prozessen der Lunge.

11. Verwendung einer wasserstoffgashaltigen und stickstoffmonoxidhaltigen Zusammensetzung zur Herstellung eines Medikamentes zur Behandlung oder Prävention der Krankheiten Pneumonie (Lungenentzündung), Lungentrauma, Asthma bronchiale, ARDS (schwere Schocklunge; Acute Respiratory Distress Syndrome), PPHN (angeborener Hochdruck im arteriellen Lungenstromgebiet; Persistent Pulmonary Hypertension of the Newbom), COPD (chronisch obstruktive Atemwegserkrankung; Chronic Obstructive Pulmonary Disease), Bronchitis (Entzündung der Bronchien), hypoxisch bedingte Vasokonstriktion (Gefäßkrampf durch Sauerstoffmangel), Fettembolie in der Lunge (Verstopfung der Lungenarterien), akute pulmonale Ödeme (akute Flüssigkeitseinlagerung in der Lunge), akute Höhenerkrankung (acute mountain sickness), erhöhter Lungenhochdruck nach herzchirurgischen Eingriffen (postcardiac surgery acute pulmonary hypertension), Aspirationssyndrom der Neugeborenen (perinatal aspiration syndrome), hyaline Membranerkrankung (hyaline membrane disease), akute Lungenembolie (acute pulmonary thromboembolism), Heparin-Protamin-Reaktionen, Sepsis, Asthmaanfall (status asthmaticus), Sauerstoffunterversorgung (hypoxia), chronischer Lungenhochdruck (chronic pulmonary hypertension), Fehlentwicklung der Lunge/Bronchien (bronchopulmonary dysplasia), chronische Lungenembolie (chronic pulmonary thromboembolism), idiopathischer oder primärer Lungenhochdruck (idiopathic pulmonary hypertension or primary pulmonary hypertension) oder chronische Sauerstoffunterversorgung (chronic hypoxia).

12. Verwendung eines deuteriumhaltigen Gasgemisches zur Herstellung eines Medikamentes zur Behandlung oder zur Prävention von Krebs.

13. Verwendung eines deuteriumhaltigen Gasgemisches zur Herstellung eines Medikamentes zur Behandlung oder Prävention von Lungenkrebs.

14. Verwendung eines wasserstoffgashaltigen Gasgemisches zur Begasung von Zellkulturen.

15. Verwendung eines wasserstoffgashaltigen Gasgemisches oder einer wasserstoffgashaltigen Zusammensetzung zur Herstellung eines Medikamentes zur Senkung der Konzentration von Peroxynitrit oder Hydroxylradikalen im Körper von Säugetieren oder dem Menschen.

16. Verwendung eines Gasgemisches mit Wasserstoffgas, Helium und Sauerstoffgas zur Herstellung eines inhalierbaren Medikamentes zur Behandlung eines gestörten Gasaustausches in der Lunge und zur Behandlung von Verschlüssen der Atemwege.

17. Verwendung einer kohlenhydrathaltigen, lipidhaltigen, peptidhaltigen oder proteinhaltigen Flüssigkeit zur Herstellung eines wasserstoffgashaltigen Medikamentes.

## Claims

1. Medicinal product comprising hydrogen gas or a source of hydrogen gas.

2. Medicinal product according to Claim 1, **characterized in that** it is gaseous or is in liquid, gel-like or solid form.

3. Medicinal product according to Claim 1 or 2, **characterized in that** the hydrogen gas comprises one or more of the hydrogen isotopes protium, deuterium or tritium.

4. Medicinal product according to any of Claims 1 to 3, comprising nitrogen monoxide, carbon monoxide, dinitrogen oxide, acetylene or ethylene.

5. Medicinal product according to any of Claims 1 to 4, comprising a gas mixture containing hydrogen gas with 1 ppm (v/v) to 99 % by volume nitrogen monoxide, carbon monoxide, dinitrogen oxide, acetylene or ethylene.

6. Medicinal product according to any of Claims 1 to 5, **characterized in that** the medicinal product containing hydrogen gas is used as inhalable gas, in the form of suppositories, ointments, solutions, dispersions, emulsions, microdroplets, microbubbles, liposomes, microparticles, aerosols, foams, particulate agents, pills, pastilles, capsules, microcapsules, chewing gum types, in carriers or as constituent of a plaster.

7. Medicinal product according to any of Claims 1 to 5, **characterized in that** the medicament comprises microdroplets, microbubbles, liposomes, microparticles or clathrates containing hydrogen gas or containing hydrogen gas and one or more of the gases nitrogen monoxide, carbon monoxide, dinitrogen oxide, acetylene or ethylene.

8. Use of a composition or gas mixture containing hydrogen gas for producing a medicinal product for the treatment or prevention of ARDS (adult respiratory distress syndrome), bronchial asthma, COPD (chronic obstructive pulmonary disease; chronic obstructive respiratory tract disorder), bronchitis, pneumonia, lung trauma, hypoxia-related vasoconstriction, for the treatment of respiratory tract occlusions (atelectases) or inflammatory processes.

9. Use of a composition or gas mixture containing hydrogen gas and containing nitrogen monoxide for producing a medicinal product for the treatment or prevention of pulmonary vasoconstriction, bronchio-constriction or respiratory tract occlusions.

10. Use of a composition containing hydrogen gas and containing nitrogen monoxide for producing a medicinal product for the treatment of reversible and irreversible pulmonary vasoconstriction (pulmonary vasospasm), bronchial constriction (spasmodic constriction of the bronchi) and inflammatory processes in the lung.

11. Use of a composition containing hydrogen gas and containing nitrogen monoxide for producing a medicinal product for the treatment or prevention of the diseases pneumonia (inflammation of the lungs), lung trauma, bronchial asthma, ARDS (acute respiratory distress syndrome; severe shock lung), PPHN (persistent pulmonary hypertension of the newborn; inborn arterial hypertension in the pulmonary circulation), COPD (chronic obstructive pulmonary disease; chronic obstructive respiratory tract disorder), bronchitis (inflammation of the bronchi), hypoxia-related vasoconstriction (vasospasm due to oxygen deficiency), fat embolism in the lung (blockage of the pulmonary arteries), acute pulmonary edema (acute fluid infiltration in the lung), acute altitude sickness (acute mountain sickness), increased pulmonary hypertension after cardiac surgery (postcardiac surgery acute pulmonary hypertension), aspiration syndrome of the newborn (perinatal aspiration syndrome), hyaline membrane disease, acute pulmonary embolism (acute pulmonary thromboembolism), heparin-protamine reactions, sepsis, asthma attack (status asthmaticus), deficient supply of oxygen (hypoxia), chronic pulmonary hypertension, faulty development of the lung/bronchi (bronchopulmonary dysplasia), chronic pulmonary embolism (chronic pulmonary thromboembolism), idiopathic or primary pulmonary hypertension or chronically deficient supply of oxygen (chronic hypoxia).

12. Use of a deuterium-containing gas mixture for producing a medicinal product for the treatment or prevention of cancer.

13. Use of a deuterium-containing gas mixture for producing a medicinal product for the treatment or prevention of lung cancer.

14. Use of a gas mixture containing hydrogen gas for gassing cell cultures.

15. Use of a gas mixture containing hydrogen gas or of a composition containing hydrogen gas for producing a medicinal product for reducing the concentration of peroxynitrite or hydroxyl free radicals in the body of mammals or humans.

16. Use of a gas mixture with hydrogen gas, helium and oxygen gas for producing an inhalable medicinal product for the treatment of disturbed gas exchange in the lung and for the treatment of respiratory tract occlusions.

17. Use of a carbohydrate-containing, lipid-containing, peptide-containing or protein-containing liquid for producing a medicinal product containing hydrogen gas.

## Revendications

1. Médicament contenant de l'hydrogène gazeux ou une source d'hydrogène gazeux.

2. Médicament selon la revendication 1,
**caractérisé en ce qu'**
il est gazeux, liquide, gélatineux ou solide.

3. Médicament selon la revendication 1 ou 2,
**caractérisé en ce que**
l'hydrogène gazeux contient un ou plusieurs des isotopes de l'hydrogène protium, deutérium ou tritium.

4. Médicament selon l'une des revendications 1 à 3, contenant du monoxyde d'azote, du monoxyde de carbone, du protoxyde d'azote, de l'acétylène ou de l'éthylène.

5. Médicament selon l'une des revendications 1 à 4, contenant un mélange de gaz contenant de l'hydrogène gazeux avec 1 ppm (v/v) à 99 % en volume de monoxyde d'azote, de monoxyde de carbone, de protoxyde d'azote, d'acétylène ou d'éthylène.

6. Médicament selon l'une des revendications 1 à 5,
**caractérisé en ce que**
le médicament contenant de l'hydrogène gazeux est utilisé comme gaz inhalable, sous forme de suppositoires, de pommades, de solutions, de dispersions, d'émulsions, de microgouttelettes de microbulles, de liposomes, de microparticules, d'aérosols, de mousses, d'agents particulaires, de pilules, de pastilles, de gélules, de gommes à mâcher, dans des excipients ("carrier") ou comme constituant d'un emplâtre.

7. Médicament selon l'une des revendications 1 à 5,
**caractérisé en ce que**
des microgouttelettes, des microbulles, des liposomes, des microparticules ou des clathrates contenant de l'hydrogène gazeux ou de l'hydrogène gazeux et un ou plusieurs des gaz monoxyde d'azote, monoxyde de carbone, protoxyde d'azote, acétylène ou éthylène, sont contenus dans le médicament.

8. Utilisation de la composition ou du mélange de gaz contenant de l'hydrogène gazeux pour la préparation d'un médicament pour le traitement ou la prévention de SDRA (Adult Respiratory Distress Syndrome), d'asthme bronchial, de MPOC (maladie pulmonaire obstructive chronique, Chronic Obstructive Pulmonary Disease), de bronchite, de pneumonie, de traumatisme pulmonaire, de vasoconstriction hypoxique, pour le traitement d'obstructions des voies respiratoires (atélectases) ou de processus inflammatoires.

9. Utilisation d'une composition contenant de l'hydrogène gazeux et du monoxyde d'azote pour la préparation d'un médicament destiné au traitement ou à la prévention de vasoconstriction pulmonaire, de broncho-constriction ou d'obstructions des voies respiratoires.

10. Utilisation d'une composition contenant de l'hydrogène gazeux et du monoxyde d'azote pour la préparation d'un médicament destiné au traitement de vasoconstrictions réversibles et irréversibles (spasmes des vaisseaux pulmonaires), de bronchoconstrictions (resserrement spastique des bronches) et de processus inflammatoires des poumons.

11. Utilisation d'une composition contenant de l'hydrogène gazeux et du monoxyde d'azote pour la préparation d'un médicament destiné au traitement ou à la prévention des maladies suivantes : pneumonie (inflammation des poumons), traumatisme pulmonaire, asthme bronchial, SDRA (syndrome de détresse respiratoire aiguë, ARDS - Acute Respiratory Distress Syndrome), HAPPN (hypertension artérielle persistante du nouveau-né, PPHN - Persistent Pulmonary Hypertension of the Newborn), MPOC (maladie pulmonaire obstructive chronique, CPOD - Chronic Obstructive Pulmonary Disease), bronchite (inflammation des bronches), vasoconstriction hypoxique (spasmes des vaisseaux par manque d'oxygène), embolies graisseuses dans les poumons (obstruction des artères pulmonaires), oedème pulmonaire aigu (accumulation aiguë de liquide dans les poumons), mal des montagnes aigu (acute mountain sickness), hypertension pulmonaire après intervention de chirurgie cardiaque (postcardiac surgery acute pulmonary hypertension), syndrome néonatal d'aspiration (perinatal aspiration syndrome), maladie des membranes hyalines (hyaline membrane disease), embolie pulmonaire aiguë (acute pulmonary thromboembolism), réactions à l'héparine - protamine, sepsis, crise d'asthme (status asthmaticus), sous-alimentation en oxygène (hypoxie), hypertension pulmonaire chronique (chronic pulmonary hypertension), dysplasie des poumons/bronches (bronchopulmonary dysplasia), embolie pulmonaire chronique (chronic pulmonary thromboembolism), hypertension pulmonaire idiopathique ou primaire (idiopathic pulmonary hypertension ou primary pulmonary hypertension) ou sous-alimentation chronique en oxygène (chronic hypoxia).

12. Utilisation d'un mélange de gaz contenant du deutérium pour la préparation d'un médicament destiné au traitement ou à la prévention du cancer.

13. Utilisation d'un mélange de gaz contenant du deutérium pour la préparation d'un médicament destiné au traitement ou à la prévention du cancer du poumon.

14. Utilisation d'un mélange de gaz contenant de l'hydrogène gazeux comme atmosphère pour des cultures de cellules.

15. Utilisation d'un mélange de gaz contenant de l'hydrogène gazeux ou d'une composition contenant de l'hydrogène gazeux pour la préparation d'un médicament destiné à abaisser la concentration de peroxynitrite ou de radicaux hydroxy dans le corps de mammifères ou d'humains.

16. Utilisation d'un mélange de gaz comportant de l'hydrogène gazeux, de l'hélium et de l'oxygène gazeux pour la préparation d'un médicament inhalable destiné au traitement d'un métabolisme gazeux perturbé dans les poumons et au traitement d'obstructions des voies respiratoires.

17. Utilisation d'un liquide contenant des hydrates de carbone, des lipides, des peptides ou des protéines pour la préparation d'un médicament contenant de l'hydrogène gazeux.
